# EUROPÄISCHE PATENTSCHRIFT

(11) **EP 0 179 408 B1**
(45) Veröffentlichungstag und Bekanntmachung des Hinweises auf die Patenterteilung: **08.09.1993**
(21) Anmeldenummer: 85113222.5
(22) Anmeldetag: 18.10.1985
(51) Int. Cl.: C07D 251/70

(54) **Neue Amidoalkylmelamine und Aminoalkylmelamine und Verfahren zu ihrer Herstellung**
Amidoalkyl melamines and aminoalkyl melamines, and process for their preparation
Amidoalkylmélamines et aminoalkylmélamines et procédé pour leur préparation

(30) Priorität: 23.10.1984 DE 3438694
(43) Veröffentlichungstag der Anmeldung: 30.04.1986
(73) Patentinhaber: BASF Aktiengesellschaft, 67063 Ludwigshafen (DE)
(72) Erfinder: Ebel, Klaus, Dr., D-6700 Ludwigshafen (DE); Reuther, Wolfgang, Dr., D-6900 Heidelberg (DE)

(56) Entgegenhaltungen:
- EP-A- 0 109 023
- DE-A- 2 038 182
- GB-A- 867 279
- A.R. KATRITZKY et al.: "Comprehensive heterocyclic chemistry", Band 3, Teil 2B, 1984, Seiten 482-483, Pergamon Press, Oxford, GB
- JOURNAL OF MEDICINAL CHEMISTRY, Band 10, Nr. 3, Mai 1967, Seiten 457-461; A.B. BORKOVEC et al.: "Insect chemosterilants. V. Derivatives of melamine"
- J. Am.Chem.Soc. 73, 2984-86, 1973
- J.Am.Chem.Soc 67, 662-64, 1945

## Beschreibung

Die vorliegende Erfindung betrifft ein Verfahren zur Herstellung von Amidoalkylmelaminen und Aminoalkylmelaminen der allgemeinen Formel I 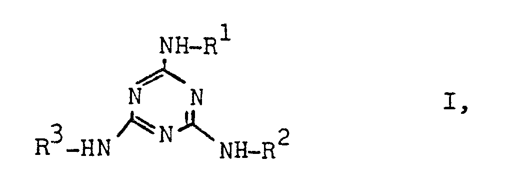
in der R¹ eine Amidoalkylgruppe -(CH₂)ₙ-NHZ oder eine Aminoalkylgruppe -(CH₂)ₙ-NH₂ bedeutet, worin n die Zahlen 2 bis 10, vorzugsweise 2 bis 6 einschließt und Z für eine Aminoschutzgruppe steht und in der die Reste R² und R³ unabhängig voneinander entweder Wasserstoff sind oder die für R¹ genannte Bedeutung haben und so erhaltene neue Amido- und Aminoalkylmelamine.

N-Alkyl- und N-Aryl-substituierte Melamine lassen sich nach Kaiser et al. J. Am. Chem. Soc. 73 (1951), 2984-86 durch Umsetzung von Cyanurchlorid bzw. Dichloraminotriazin bzw. Diaminochlortriazin darstellen. Auf ähnliche Weise sind 2,4,6-Tris(alkanol-substituierte amino)-Triazine hergestellt worden (US-A 3 879 389).

Von den erfindungsgemäßen erhältlichen Aminoalkylmelaminen sind bisher nur N-Mono-(2-aminoethyl)melamin und N-Mono-(6-aminohexyl)melamin beschrieben. Sie wurden durch Umsetzung von Diaminochlortriazin mit den entsprechenden Alkylendiaminen hergestellt.

N-Mono-(2-aminoethyl)melamin wurde in J. Med. Chem. 10, 457-461 (1967) ohne Angabe einer Herstellvorschrift als Hydrochlorid mit Kristallwasser nur durch eine Elementaranalyse charakterisiert, weshalb angenommen wird, daß kein oder zumindest kein reines N-Mono-(2-aminoethyl)melamin erhalten wurde.

N-Mono-(6-aminohexyl)melamin wurde in J. Am. Chem. Soc. 67, 662-664 (1945) ohne Angabe einer Herstellvorchrift mit einem Schmelzpunkt von 154°C charakterisiert, während die Herstellung in GB 867 279 beschrieben wird. Der dort angegebene Schmelzpunkt liegt bei 175-177°C.
Zi/P
N,N'-Bis und N,N',N''-Tris-aminoalkylmelamine waren demgegenüber bisher nicht bekannt. Dies liegt offensichtlich daran, daß die Umsetzung von Chlortriazinen oder Aryloxytriazinen mit Alkylendiaminen bevorzugt zu polymeren Produkten führt (US-A 2 545 049).

Der Erfindung lag somit die Aufgabe zugrunde, einen neuen Weg zu finden, der es gestattet, die Aminoalkylmelamine synthetisch zugänglich zu machen und neben den N-Mono-(aminoalkyl)melaminen auch die N,N'-Bis- und N,N',N''-Tris-(aminoalkyl)melamine herzustellen.

Demgemäß wurde ein Verfahren zur Herstellung von Amidoalkylmelaminen und Aminoalkylmelaminen der allgemeinen Formel I 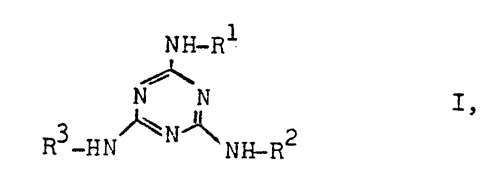
in der R¹ eine Amidoalkylgruppe -(CH₂)ₙ-NHZ oder eine Aminoalkylgruppe -(CH₂)ₙ-NH₂ bedeutet, worin n die Zahlen 2 bis 10, vorzugsweise 2 bis 6 einschließt und Z für eine Aminoschutzgruppe steht und in der die Reste R² und R³ unabhängig voneinander entweder Wasserstoff sind oder die für R¹ genannte Bedeutung haben, gefunden, gemäß dem man Chlortriazine mit Diaminen der allgemeinen Formel H₂N-(CH₂)ₙ-NHZ, in der n und Z die obengenannte Bedeutung haben, zu Amidoalkylmelaminen umsetzt und diese gegebenenfalls durch Abspaltung der Gruppe Z in die Aminoalkylmelamine überführt.

Das Syntheseprinzip wird durch folgendes Formelschema wiedergegeben: 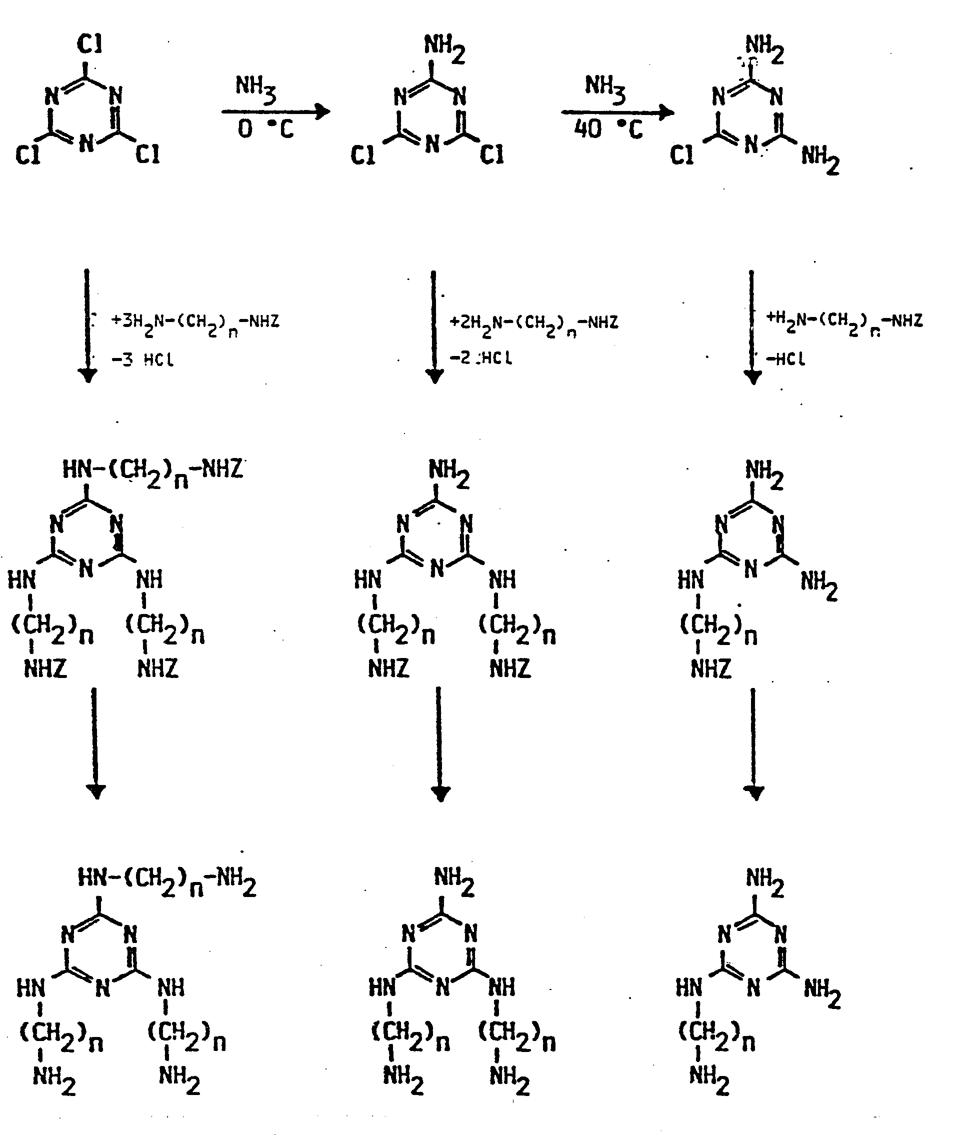
Zur Darstellung der Amidoalkylmelamine geht man von Cyanurchlorid bzw. dem daraus durch Umsetzung mit Ammoniak erhältlichen Dichloraminotriazin oder Diaminochlortriazin aus. Alle drei Edukte sind aus J. Am. Chem. Soc. 73 (1951), 2984 bekannt und werden in ähnlicher Weise wie dort für Alkylamine beschrieben mit einseitig durch eine Schutzgruppe Z blockierten Alkylendiaminen zu den Amidoalkylmelaminen umgesetzt.

Zum Schutz der Aminogruppe sind neben den Formyl- oder Tosylrest insbesondere die Acetylgruppe, die gegebenenfalls noch durch Halogenatome z.B. Fluor oder Chlor substituiert sein kann, und Gruppen der Formel 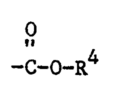
geeignet, in denen R⁴ ein verzweigter oder unverzweigter Alkylrest mit 1 bis 15, insbesondere 1 bis 6 Kohlenstoffatomen, ein Cycloalkylrest mit 5 bis 8 Kohlenstoffatomen, ein Arylrest, der ggf. noch Alkylsubstituenten mit 1 bis 8, insbesondere 1 bis 4 Kohlenstoffatomen tragen kann, oder ein Aralkylrest mit 7 bis 12 Kohlenstoffatomen sein kann. Die für R⁴ genannten Reste können noch mit unter den Reaktionsbedingungen inerten Gruppen z.B. die Nitro-, Halogen- oder Alkoxygruppe substituiert sein.

Als Aminoschutzgruppen sind insbesondere die Acetyl-, Trifluoracetyl-, Methoxy-, Ethoxy-, tert. Butoxy-, Cyclopentyloxy- und Phenoxycarbonylgruppe sowie die Carbobenzoxy- oder para-Nitrobenzoxygruppe zu nennen.

Die Umsetzung der Chlortriazine mit den einseitig geschützten Alkylendiaminen -H₂N-(CH₂)ₙ-NHZ, wobei n die Zahlen 2 bis 10, insbesondere 2 bis 6 einschließt, kann in organischen Lösungsmitteln z.B. Dioxan erfolgen, vorteilhaft ist es jedoch, in wäßriger Lösung zu arbeiten.

Die einseitig geschützten Alkylendiamine werden in der Regel im Überschuß zu den entsprechenden Chlortriazinen zugesetzt, dabei hat sich ein Verhältnis von 1 zu 1,1 bis 1 zu 1,35 pro auszutauschendes Chloratom bewährt. 1 mol Cyanurchlorid wird also vorzugsweise mit 3,3 bis 4,1 mol Alkylendiamin, 1 mol Dichloraminotriazin vorzugsweise mit 2,2 bis 2,7 mol Alkylendiamin und 1 mol Diaminochlortriazin vorzugsweise mit 1,1 bis 1,4 und insbesondere mit 1,1 bis 1,2 mol Alkylendiamin umgesetzt.

Um die bei der Reaktion entstehende Salzsäure abzufangen, wird eine Base z.B. Kaliumcarbonat, Natriumcarbonat oder Natronlauge zugesetzt. Hierbei ist darauf zu achten, daß die Base nicht im Überschuß verwendet wird, da sonst eine Spaltung der Säureamidfunktion stattfindet. Aus diesem Grund ist es vorteilhaft, die Base allmählich zuzugeben, so daß der pH-Wert bei ca. 8,0 gehalten wird.

Bis zur Beendigung der Reaktion, d.h. bis keine Base mehr verbraucht wird, wird unter Rückfluß gekocht.

Die Aufarbeitung und Reinigung des Produktes erfolgt in an sich bekannter Weise, indem man das aus der wäßrigen Lösung ausfallende Amidoalkylmelamin absaugt und in einem geeigneten Lösungsmittel, in der Regel Wasser, Ethanol oder einem Ethanol/Wasser-Gemisch umkristallisiert.

Zur Überführung in die Aminoalkylmelamine wird die Aminoschutzgruppe nach literaturbekannten Methoden, z.B. durch katalytische Hydrierung im Fall der Benzylcarbamate oder durch alkalische Hydrolyse abgespalten.

Bei der Hydrolyse ist darauf zu achten, daß die Reaktionszeiten möglichst kurz gehalten werden, damit die direkt mit dem Triazinring verbundene NH-Funktion nicht angegriffen wird.

Die erfindungsgemäßen Amidoalkylmelamine und Aminoalkylmelamine sind wertvolle Zwischenprodukte für organische Synthesen, z.B. für Polyisocyanat-Polyadditionsreaktionen.

Die nachfolgenden Beispiele sollen die Erfindung näher erläutern.

Die ¹H-NMR-Spektren der Verbindungen sind in DMSO-d₆ bei 60 MHz aufgenommen worden, die chemischen Verschiebungen δ beziehen sich auf Tetramethylsilan als internen Standard. Für die Art der Signale werden folgende Abkürzungen gewählt: s = Singulett, d = Dublett, t = Triplett, q = Quartett, m = Multiplett, br = breites Signal.

### Beispiel 1

### N,N',N''-Tris-(6-acetamidohexyl)melamin

18,4 g (0,1 mol) Cyanurchlorid, 56,0 g (0,35 mol) Mono-N-acetylhexamethylendiamin und 41,5 g (0,3 mol) wasserfreies Kaliumcarbonat in 450 ml Dioxan wurden 5 Stunden unter Rühren und Rückfluß gekocht. Dann wurde heiß filtriert und das Filtrat vom Lösungsmittel befreit. Der Rückstand wurde mit 0,5 l Wasser gerührt und das Produkt abgesaugt und getrocknet. Man erhielt 40 g (73 %) der Tris-Verbindung als farblose Kristalle. Der Schmelzpunkt beträgt 215°C nach Umkristallisieren aus Ethanol/Wasser.

### ¹H-NMR

- δ [ppm] =: 1,3 (s, br., 24H) 1,8 (s, 9H) 3,2 (s, br., 12H) 3,6 (s, br., 3H) 7,8 (s,br.,3H)

| Analyse: | | | |
|---|---|---|---|
| | C | H | N |
| Ber.: | 58,99 | 9,35 | 22,93 |
| Gef.: | 58,2 | 9,2 | 22,2 |

### Beispiel 2

### N,N'-Bis-(6-acetamidohexyl)melamin

Zur Suspension von 41,0 g (0,25 mol) Dichloraminotriazin in 300 ml Wasser wurden 87,0 g (0,55 mol) Mono-N-acetylhexamethylendiamin gegeben und zum Sieden erhitzt. Durch sukzessive Zugabe von gesättigter Natriumcarbonatlösung wurde der pH-Wert bei 8,0 gehalten. Nach 2 Stunden war die Reaktion beendet. Man ließ abkühlen, saugte das Produkt ab und wusch mit Wasser chloridfrei. Nach dem Trocknen erhielt man 95 g (93 %) farblose Kristalle, die nach Umkristallisieren aus Ethanol/Wasser einen Schmelzpunkt von 126°C zeigten.

### ¹H-NMR

- δ [ppm] =: 1,3 (s, br., 16H) 1,8 (s, 6H) 3,2 (m, 8H) 6,0 (s, br., 2H) 6,4 (s, br., 2H) 7,8 (s, br., 2H)

| Analyse: | | | |
|---|---|---|---|
| | C | H | N |
| Ber.: | 53,86 | 8,88 | 27,43 |
| Gef.: | 55,3 | 8,7 | 27,2 |

### Beispiel 3

### N-Mono-(6-acetamidohexyl)melamin

Man brachte 36,5 g (0,25 mol) Diaminochlortriazin und 46,8 g (0,30 mol) Mono-N-acetylhexamethylendiamin in 300 ml Wasser analog wie in Beispiel 2 zur Reaktion und arbeitete analog auf. Nach Umkristallisieren aus Wasser erhielt man 62 g (93 %) farblose Kristalle vom Schmp. 94 - 96°C.

### ¹H-NMR

- δ [ppm] =: 1,3 (s, br., 8H) 1,8 (s, 3H) 3,1 (m, 4H) 6,1 (s, br., 4H) 6,5 (s, br., 1H) 7,8 (s, br., 1H)

| Analyse: | | | |
|---|---|---|---|
| | C | H | N |
| Ber.: | 49,42 | 7,92 | 36,68 |
| Gef.: | 48,2 | 7,7 | 35,1 |

### Beispiel 4

### N,N',N''-Tris-(2-ethoxycarbonylamidoethyl)melamin

Zur Suspension von 92,5 g (0,5 mol) Cyanurchlorid in 500 ml Wasser tropfte man 218,0 g (1,65 mol) Ethylendiamin-N-mono-carbamidsäureethylester. Dann wurde langsam erwärmt und durch sukzessive Zugabe von 25%iger Natronlauge auf pH 8,1 gehalten. Zum Schluß wurde unter Rückfluß gekocht. Sobald keine Lauge mehr verbraucht wurde, ließ man abkühlen und saugte das Produkt ab. Nach Umkristallisieren aus Ethanol/Wasser erhielt man 197 g (84 %) farblose Kristalle vom Schmp. 148°C.

### ¹H-NMR

- δ [ppm] =: 1,2 (t, 9H) 3.2 (m, 12H) 4,0 (q, 6H) 7,1 (s, br., 3H) 7,7 (s,br.,3H)

| Analyse: | | | |
|---|---|---|---|
| | C | H | N |
| Ber.: | 45,85 | 7,05 | 26,74 |
| Gef.: | 45,5 | 6,9 | 26,6 |

### Beispiel 5

### N,N'-Bis-(2-ethoxycarbonylamidoethyl)melamin

Analog Beispiel 4 wurden 33 g (0,2 mol) Dichloraminotriazin und 58 g (0,44 mol) Ethylendiamin-N-mono-carbamidsäureethylester in 160 ml Wasser zur Reaktion gebracht und aufgearbeitet Nach Umkristallisieren aus Ethanol/Wasser erhielt man 58 g (81 %) farblose Kristalle vom Schmp. 146°C.

### ¹H-NMR

- δ [ppm] =: 1,2 (t, 6H) 3,2 (m, 8H) 4,0 (q, 4H) 6,1 (s, br., 2H) 6,4 (s, br., 2H) 7,0 (s, br., 2H)

| Analyse: | | | |
|---|---|---|---|
| | C | H | N |
| Ber.: | 43,81 | 6,79 | 31,44 |
| Gef.: | 43,6 | 6,7 | 31,4 |

### Beispiel 6

### N-Mono-(2-ethoxycarbonylamidoethyl)melamin

Analog Beispiel 4 wurden 43,8 g (0,3 mol) Diaminochlortriazin und 47,4 g (0,36 mol) Ethylendiamin-N-mono-carbamidsäureethylester in 270 ml Wasser zur Reaktion gebracht und aufgearbeitet. Nach Umkristallisieren aus Wasser erhielt man 58,2 g (80 %) farblose Kristalle vom Schmp. 198°C.

### ¹H-NMR

- δ [ppm] =: 1,2 (t, 3H) 3,2 (m, 4H) 4,0 (q, 2H) 6,1 (s, br., 4H) 6,4 (s, br., 1H) 7,1 (s, br., 1H)

| Analyse: | | | |
|---|---|---|---|
| | C | H | N |
| Ber.: | 39,83 | 6,27 | 40,64 |
| Gef.: | 39,3 | 6,1 | 39,8 |

### Beispiel 7

### N,N',N''-Tris-(6-benzoxycarbonylamidohexyl)melamin

Analog Beispiel 4 wurden 1,85 g (0,01 mol) Cyanurchlorid und 10,0 g (0,04 mol) Mono-N-Carbobenzoxyhexamethylendiamin in 100 ml Wasser zur Reaktion gebracht und aufgearbeitet. Nach Umkristallisieren aus Ethanol erhielt man 5,2 g (63 %) farblose Kristalle vom Schmp. 98°C.

### ¹H-NMR

- δ [ppm] =: 1,3 (s, br., 24H) 3,1 (m, 12H) 5,0 (s, 6H) 6,4 (s, br., 3H) 7,2 und 7,3 (m und s, 18H)

| Analyse: | | | |
|---|---|---|---|
| | C | H | N |
| Ber.: | 65,43 | 7,69 | 15,26 |
| Gef.: | 64,9 | 7,2 | 15,3 |

### Beispiel 8

### N,N'-Bis-(6-benzoxycarbonylamidohexyl)melamin

Analog Beispiel 4 wurden 5,8 g (0,035 mol) Dichloraminotriazin und 23,0 g (0,092 mol) Mono-N-carbobenzoxyexamethylendiamin in 170 ml Wasser zur Reaktion gebracht und aufgearbeitet. Nach Umkristallisieren aus Ethanol/Wasser 2 : 1 erhielt man 18,9 g (91 %) farblose Kristalle vom Schmp. 101°C.

### ¹H-NMR

- δ [ppm] =: 1,3 (s, br., 16H) 3,1 (m, 8H) 5,0 (s, 4H) 5,9 (s, br., 2H) 6,3 (s, br., 2H) 7,2 und 7,3 (m und s, 12H)

| Analyse: | | | |
|---|---|---|---|
| | C | H | N |
| Ber.: | 62,82 | 7,48 | 18,91 |
| Gef.: | 62,8 | 7,1 | 18,6 |

### Beispiel 9

### N-Mono-(6-benzoxycarbonylamidohexyl)melamin

Wie in Beispiel 4 beschrieben, wurden 7,3 g (0,050 mol) Diaminochlortriazin und 14,0 g (0,056 mol) Mono-N-carbobenzoxyhexamethylendiamin in 100 ml Wasser zur Reaktion gebracht. Nach dem Abkühlen schied sich ein fast farbloses Öl ab. Man trennte die Ölphase ab und extrahierte die Wasserphase mit Essigester. Dann wurden die vereinigten organischen Phasen mit Wasser gewaschen, über Natriumsulfat getrocknet und im Vakuum das Lösungsmittel entfernt. Man erhielt 17,3 g (96 %) eines farblosen Öls, das spektroskopisch (¹H-NMR, ¹³C-NMR, IR, MS) eindeutig als N-Mono-(6-benzoxycarbonylamidohexyl)melamin identifiziert wurde.

### ¹H-NMR

- δ [ppm] =: 1,3 (s, br., 8H) 3,1 (m, 4H) 5,0 (s, 2H) 6,3 (m, 5H) 7,2 und 7,3 (m und s, 6H)

| Analyse: | | | |
|---|---|---|---|
| | C | H | N |
| Ber.: | 56,81 | 7,01 | 27,28 |
| Gef.: | 57,4 | 7,1 | 24,6 |

### Beispiel 10

### N,N',N''-Tris-(2-aminoethyl)melamin

Man kochte 25,5 g (0,05 mol) N,N',N''-Tris-(2-ethoxycarbonylamidoethyl)melamin (aus Beisp. 4) 8 h in einer Lösung von 36 g (0,90 mol) NaOH in 300 ml Wasser. Es entstand eine klare Lösung. Die Natronlauge wurde mit 43,0 g (0,45 mol) konz. Schwefelsäure neutralisiert und das Lösungsmittel abgezogen. Der feste farblose Rückstand wurde mit Ethanol kontinuierlich extrahiert. Nach da Entfernen des Ethanols im Vakuum erhielt man 10 g (78 %) eines schwach gelben Öls, welches ¹H-NMR-, ¹³C-NMR- und IR-spektroskopisch eindeutig als N,N',N''-Tris-(2-aminoethyl)melamin identifiziert wurde.

### ¹H-NMR

- δ [ppm] =: 1,9 (s, br., 6H) 2,6 (t, 6H) 3,1 (t, 6H) 6,6 (s, br., 3H)

| Analyse: | | | |
|---|---|---|---|
| | C | H | N |
| Ber.: | 42,34 | 8,29 | 49,37 |
| Gef.: | 42,5 | 8,5 | 47,9 |

### Beispiel 11

### N,N'-Bis-(2-aminoethyl)melamin

Man hydrolysierte, wie in Beispiel 10 beschrieben, 54,0 g (0,15 mol) N,N'-Bis-(2-ethoxycarbonylamidoethyl)melamin (aus Beisp. 5) 6 h in einer Lösung von 51,0 g (1,30 mol) Natriumhydroxid in 300 ml Wasser und neutralisierte mit 61,0 g (0,65 mol) konz. Schwefelsäure. Nach analoger Aufarbeitung wie in Beispiel 10 erhielt man 25,0 g (79 %) farblose Kristalle mit den Schmp. 135°C nach Umkristallisation aus Ethanol/Ether.

### ¹H-NMR

- δ [ppm] =: 1,8 (s, br., 4H) 2,6 (t, 4H) 3,2 (t, 4H) 6,2 (s, br., 2H) 6,7 (s, br., 2H)

| Analyse: | | | |
|---|---|---|---|
| | C | H | N |
| Ber.: | 39,61 | 7,60 | 52,79 |
| Gef.: | 39,8 | 7,5 | 52,3 |

### Beispiel 12

### N-Mono-(2-aminoethyl)melamin

Man hydrolisierte, wie in Beispiel 10 beschrieben, 60 g (0,25 mol) N-Mono-(2-ethoxycarbonylamidoethyl)melamin (aus Beisp. 6) 3 h in einer Lösung von 40 g (1,0 mol) Natriumhydroxid in 300 ml Wasser und engte auf etwa 70 ml ein. Nach dem Abkühlen wurde der ausgefallene Niederschlag abgesaugt und mehrmals mit Ethanol ausgekocht und abfiltriert. Die Ethanolfiltrate wurden zur Trocknung eingeengt und der so erhaltene Rückstand aus Wasser umkristallisiert. Man erhielt 28 g (66 %) farblose Kristalle von Schmp. 112°C.

### ¹H-NMR

- δ [ppm] =: 2,3 (s, br., 2H) 2,6 (t, 2H) 3,2 (t, 2H) 6,4 (s, br., 4H) 6,8 (s, br., 1H)

| Analyse: | | | |
|---|---|---|---|
| | C | H | N |
| Ber.: | 35,50 | 6,55 | 57,95 |
| Gef.: | 36,0 | 6,5 | 57,9 |

### Beispiel 13

### N-Mono-(6-aminohexyl)melamin

Man hydrierte 7,2 g (0,02 mol) N-Mono-(6-benzoxycarbonylamidohexyl)melamin (aus Beisp. 9) 20 h bei 30 bar Wasserstoffdruck und Raumtemperatur in einer Mischung aus 100 ml Wasser und 100 ml Eisessig mit 0,2 g 10 % Palladium auf Aktivkohle als Katalysator. Dann wurde der Katalysator abfiltriert und zur Trockne im Vakuum eingedampft. Den Rückstand löste man in 50 ml heißen Wasser, filtrierte nochmals und stellte mit 50%iger Natronlauge alkalisch, wobei das Produkt ausfiel. Man erhielt 2,5 g (56 %) farblose Kristalle vom Schmp. 225 bis 228°C. Es wurde aus Wasser/Ethanol 2 : 1 umkristallisiert.

### ¹H-NMR

- δ [ppm] =: 1,3 (s, br., 8H) 2,1 (s, 2H) 2,5 (m, 2H) 3,2 (m, 2H) 6,0 (s, br., 4H) 6,4 (t, 2H)

| Analyse: | | | |
|---|---|---|---|
| | C | H | N |
| Ber.: | 47,98 | 8,50 | 43,52 |
| Gef.: | 47,7 | 8,7 | 42,1 |

### Beispiel 14

### N,N'-Bis-(6-aminohexyl)melamin

Man hydrierte 17,8 g (0,03 mol) N,N'-Bis-(6-benzoxycarbonylamidohexyl)melamin (aus Beispiel 8) 20h bei 30 bar Wasserstoffdruck und Raumtemperatur in 100 ml Ethanol und 100 ml Eisessig mit 0,6 g 10 % Palladium auf Aktivkohle als Katalysator. Dann wurde der Katalysator abfiltriert und zur Trockene im Vakuum eingedampft. Der Rückstand wurde durch Chromatographie an stark basischem Ionentauscher vom noch anhaftenden Eisessig befreit. Nach dem Entfernen des Ethanols und Trocknen im Vakuum erhielt man 7,0 g (72 %) eines fast farblosen Öls.

### ¹H-NMR

- δ [ppm] =: 1,3 (s, br., 16H) 2,2 (s, 4H) 2,5 (m, 4H) 3,2 (m, 4H) 6,0 (s, br., 2H) 6,4 (s, br., 2H)

| Analyse: | | | |
|---|---|---|---|
| | C | H | N |
| Ber.: | 55,52 | 9,94 | 34,53 |
| Gef.: | 55,0 | 10,0 | 34,1 |

### Beispiel 15

### N,N',N''-Tris-(6-aminohexyl)melamin

Man hydrierte 21,2 g (0,03 mol) N,N',N''-Tris-(6-benzoxycarbonylamidohexyl)melamin (aus Beispiel 7) wie in Beispiel 14 beschrieben. Nach analoger Aufarbeitung erhielt man 11,3 g (89 %) eines fast farblosen Öls.

### ¹H-NMR

- δ [ppm] =: 1,3 (s, br., 24H) 2,0 (s, 6H) 2,5 (m, 6H) 3,2 (m, 6H) 6,3 (s, br., 3H)

| Analyse: | | | |
|---|---|---|---|
| | C | H | N |
| Ber.: | 59,54 | 10,71 | 29,76 |
| Gef.: | 59,1 | 10,8 | 29,2 |

## Patentansprüche

1. Verfahren zur Herstellung von Amidoalkylmelaminen und Aminoalkylmelaminen der allgemeinen Formel I 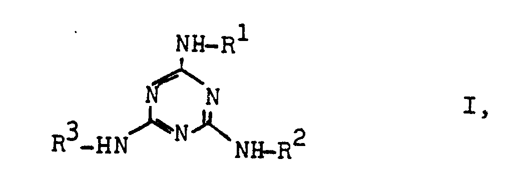 in der R¹ eine Amidoalkylgruppe -(CH₂)ₙ-NHZ oder eine Aminoalkylgruppe -(CH₂)ₙ-NH₂ bedeutet, worin n die Zahlen 2 bis 10, vorzugsweise 2 bis 6 einschließt und Z für eine Aminoschutzgruppe steht und in der die Reste R² und R³ unabhängig voneinander entweder Wasserstoff sind oder die für R¹ genannte Bedeutung haben, dadurch gekennzeichnet, daß man Chlortriazine mit Diaminen der allgemeinen Formel H₂N-(CH₂)ₙ-NHZ, in der n und Z die obengenannte Bedeutung haben, zu den Amidoalkylmelaminen umsetzt und diese gegebenenfalls durch Abspaltung der Gruppe Z in die Aminoalkylmelamine überführt.

2. Verfahren nach Anspruch 1, dadurch gekennzeichnet, daß die Gruppe Z eine Carbobenzoxygruppe 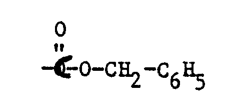 ist, und die Abspaltung dieser Gruppe Z durch katalytische Hydrierung erfolgt.

3. Verfahren nach Anspruch 1, dadurch gekennzeichnet, daß die Gruppe Z eine Acylgruppe oder eine Gruppe 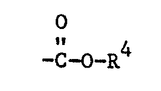 ist, in der R⁴ ein Alkyl-, Cycloalkyl-, Aryl- oder Aralkylrest -(CH₂)ₘ-C₆H₅ ist, wobei m eine Zahl 2 bis 4 bedeutet, und die Abspaltung dieser Gruppe Z durch alkalische Hydrolyse erfolgt.

4. Verfahren nach Anspruch 1, dadurch gekennzeichnet, daß man die einseitig geschützten Alkylendiamine im Überschuß zu den entsprechenden Chlortriazinen verwendet.

5. Verfahren nach Anspruch 4, dadurch gekennzeichnet, daß man 1 mol Cyanurchlorid mit 3,3 bis 4,1 mol einseitig geschütztem Alkylendiamin umsetzt.

6. Verfahren nach Anspruch 4, dadurch gekennzeichnet, daß man 1 mol Dichloraminotriazin mit 2,2 bis 2,7 mol einseitig geschütztem Alkylendiamin umsetzt.

7. Verfahren nach Anspruch 4, dadurch gekennzeichnet, daß man 1 mol Diaminochlortriazin mit 1,1 bis 1,4, vorzugsweise 1,1 bis 1,2 mol einseitig geschütztem Alkylendiamin umsetzt.

## Claims

1. A process for the preparation of amidoalkylmelamines and aminoalkylmelamines of the formula I 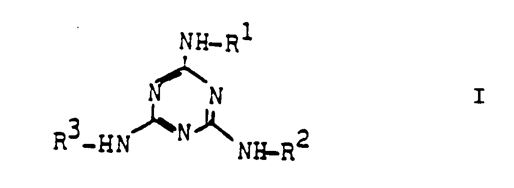 where R¹ is amidoalkyl -(CH₂)ₙ-NHZ or aminoalkyl - (CH₂)ₙ-NH₂, in which n is from 2 to 10, preferably 2 to 6, and Z is an amino-protecting group and where R² and R³, independently of each other, either are hydrogen or have the meanings mentioned for R¹, which comprises reacting chlorotriazines with diamines of the formula H₂N-(CH₂)ₙ-NHZ, in which n and Z have the abovementioned meanings, to give the amidoalkylmelamines, with or without conversion of these into the aminoalkylmelamines by elimination of the group Z.

2. A process as claimed in claim 1, wherein
Z is carbobenzoxy 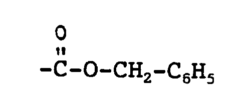 and the elimination of this group Z is carried out by catalytic hydrogenation.

3. A process as claimed in claim 1, wherein Z is acyl or 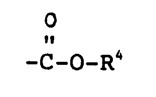 , where R⁴ is alkyl, cycloalkyl, aryl or aralkyl -(CH₂)ₘ-C₆H₅, where m is from 2 to 4, and the elimination of this group Z is carried out by alkaline hydrolysis.

4. A process as claimed in claim 1, wherein the monoprotected alkylenediamines are used in excess with respect to the corresponding chlorotriazines.

5. A process as claimed in claim 4, wherein 1 mol of cyanuric chloride is reacted with from 3.3 to 4.1 mol of monoprotected alkylenediamine.

6. A process as claimed in claim 4, wherein 1 mol of dichloroaminotriazine is reacted with from 2.2 to 2.7 mol of monoprotected alkylenediamine.

7. A process as claimed in claim 4, wherein 1 mol of diaminochlorotriazine is reacted with from 1.1 to 1.4, preferably from 1.1 to 1.2, mol of monoprotected alkylenediamine.

## Revendications

1. Procédé de préparation d'amidoalkylmélamines et d'aminoalkylmélamines de la formule générale I 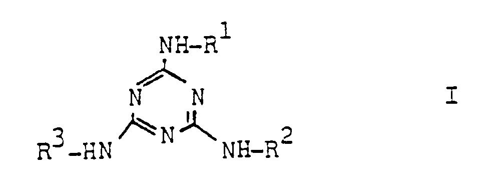 dans laquelle R¹ représente un radical amidoalkyle -(CH₂)ₙ-NHZ ou un radical aminoalkyle -(CH₂)ₙ-NH₂, n possède une valeur qui varie de 2 à 10, de préférence de 2 à 6 et Z représente un radical protégeant la fonction amino et les symboles R² et R³, indépendamment l'un de l'autre, représentent des atomes d'hydrogène ou ont les significations attribuées à R¹, caractérisé en ce que l'on fait réagir des chlorotriazines avec des diamines de la formule générale H₂N-(CH₂)ₙNHZ dans laquelle n et Z possède les significations qui leur ont été précédemment attribuées, de manière à obtenir les amidoalkylmélamines et on convertit éventuellement celles-ci en les aminoalkylmélamines par séparation du radical Z.

2. Procédé suivant la revendication 1, caractérisé en ce que le radical Z représente un groupe carbobenzoxy 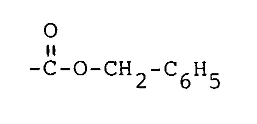 et la séparation de ce radical Z s'effectue par hydrogénation catalytique.

3. Procédé suivant la revendication 1, caractérisé en ce que le radical Z est un groupe acyle ou un groupe de la formule 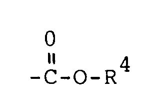 dans laquelle R⁴ représente un radical alkyle, cycloalkyle, aryle ou aralkyle -(CH₂)ₘ-C₆H₅ où m est un nombre égal à 2, 3 ou 4 et la séparation de ce radical Z s'effectue par hydrolyse alcaline.

4. Procédé suivant la revendication 1, caractérisé en ce que l'on utilise les alkylènediamines unilatéralement protégées en excès par rapport aux chlorotriazines correspondantes.

5. Procédé suivant la revendication 4, caractérisé en ce que l'on fait réagir une mole de chlorocyanure avec 3,3 à 4,1 moles d'alkylènediamine unilatéralement protégée.

6. Procédé suivant la revendication 4, caractérisé en ce que l'on fait réagir 1 mole de dichloraminotriazine avec 2,2 à 2,7 moles d'alkylènediamine unilatéralement protégée.

7. Procédé suivant la revendication 4, caractérisé en ce que l'on fait réagir 1 mole de diaminochlorotriazine avec 1,1 à 1,4 mole, de préférence 1,1 à 1,2 mole, d'alkylènediamine unilatéralement protégée.
